# EUROPÄISCHE PATENTSCHRIFT

(11) **EP 2 260 004 B1**
(45) Veröffentlichungstag und Bekanntmachung des Hinweises auf die Patenterteilung: **27.06.2018**
(21) Anmeldenummer: 09727738.8
(22) Anmeldetag: 03.04.2009
(51) Int. Cl.: C01G 39/00, C01G 53/00, C07C 51/16, B01J 23/887, B01J 27/192

(54) **VERFAHREN ZUR HERSTELLUNG VON NANOKRISTALLINEM BISMUT-MOLYBDÄNMISCHOXID**
METHOD FOR PRODUCING A NANOCRYSTALLINE BISMUTH-MOLYBDENUM MIXED OXIDE
PROCÉDÉ POUR PRODUIRE UN OXYDE MIXTE BISMUTH-MOLYBDÈNE NANOCRISTALLIN

(30) Priorität: 04.04.2008 DE 102008017308
(43) Veröffentlichungstag der Anmeldung: 15.12.2010
(73) Patentinhaber: Süd-Chemie IP GmbH & Co. KG, 81925 München (DE)
(72) Erfinder: HAGEMEYER, Alfred, 83043 Bad Aibling (DE); WEGNER, Oliver, 83052 Heufeldmühle (DE); NEUMANN, Silvia, 83109 Grosskarolinenfeld (DE); WÖLK, Hans-Jörg, 83022 Rosenheim (DE)
(74) Vertreter: Kuba, Stefan
(86) Internationale Anmeldenummer: PCT/EP2009/002475
(87) Internationale Veröffentlichungsnummer: WO 2009/121625

(56) Entgegenhaltungen:
- WO-A-2007/042369
- DE-A1-102006 032 452
- FR-A- 2 826 959

## Beschreibung

Die Erfindung betrifft ein Verfahren zur Herstellung eines nanokristallinen Bismut-Molybdänmischoxids.

Bismut-Molybdänmischoxide werden bisher im Stand der Technik durch Fällungsmethoden, Sol-Gel-Verfahren oder Festkörperreaktionen erhalten.

Die US 2007/0238904 A1 offenbart ein Bismut-Molybdänmischoxid, das durch Fällung und anschließende Kalzinierung erhalten wird. Das Bismut-Molybdänmischoxid eignet sich als Katalysator zur Umsetzung von Propen oder Isobuten zu Acrolein oder Methacrolein.

Die WO 2008/028681 und DE 10 2006 032 452 A1 offenbaren ein Verfahren zur Herstellung nanokristalliner Metalloxide oder Metall-Mischoxide. In diesen Schriften findet sich kein Hinweis, dass mit den Verfahren spezielle nanokristalline Bismut-Molybdänmischoxide hergestellt werden können, die sich als Katalysator für die Umsetzung von beispielsweise Propen zu Acrolein besonders eignen.

WO 2007/042369 A1 offenbart Mischoxidkatalysatoren für die katalytische Gasphasenoxidation von Olefinen und methylierten Aromaten, Verfahren zur Herstellung der Katalysatoren und die Umsetzung zu Aldehyden und Carbonsäuren mit Luft oder Sauerstoff in Gegenwart von inerten Gasen in unterschiedlichen Verhältnissen, bei erhöhten Temperaturen und Druck. Der Mischoxidkatalysator ist ein Molybdän-Bismut-Mischoxid mit diversen Dotierungen.

Über herkömmliche Verfahren lässt sich ein kristallines Molybdänmischoxid und somit auch ein Bismut-Molybdänmischoxid nur schwer erhalten. So offenbaren G.A. Zenkovets et. al., "The structural genesis of a complex (MoVW)5O14 oxide during thermal treatments and its redox behaviour at elevated temperatures", Materials Chemistry and Physics, 103 (2007), 295-304, dass ein über Fällung und Sprühtrocknung erhaltenes Molybdän-Mischoxid eine amorphe Struktur aufweist. Dieses Mischoxid liegt in Form von großen Aggregaten von etwa 5 µm Größe vor. Durch nachfolgendes Kalzinieren bildet sich innerhalb der Aggregate eine teilweise nanokristalline Struktur aus. Erst nach längerer thermischer Behandlung bei etwa 440 °C bildet sich eine reine kristalline Phase mit Kristalliten von mehr als 1000 nm Größe. Die Herstellung eines nanokristallinen Molybdänmischoxids lässt sich also nur schwer bewerkstelligen, insbesondere hinsichtlich der Herstellung kleiner Kristallite.

Nachteilig an den Blei-Molybdänmischoxiden des Standes der Technik ist somit insbesondere, dass keine einheitliche Partikelgröße der Molybdänmischoxide erhalten werden kann und eine Steuerung der Kristallisation, insbesondere hinsichtlich der Kristallitgröße, nicht möglich ist. Ebenso ist die BET-Oberfläche der Molybdänmischoxide des Standes der Technik meist zu gering. Gewünscht, insbesondere für katalytische Anwendungen, ist eine kleine Partikelgröße mit möglichst großer BET-Oberfläche.

Aufgabe der vorliegenden Erfindung war somit die Bereitstellung eines Verfahrens mit dem ein nanokristallines Bismut-Molybdänmischoxid erhalten werden kann, das als Katalysator bei katalytischen Umsetzungen, eine erhöhte Aktivität und Selektivität für das gewünschte Endprodukt aufweist.

Die Aufgabe wird gelöst durch ein Verfahren zur Herstellung eines nanokristallinen Bismut-Molybdänmischoxids, umfassend die Schritte
a) des Einbringens einer Lösung, Suspension oder Aufschlämmung, enthaltend eine Molybdänausgangsverbindung, eine Bismutausgangsverbindung, eine Eisenausgangsverbindungen, sowie eine Nickelausgangsverbindung und eine Zinkausgangsverbindung in eine Reaktionskammer mittels eines Trägerfluids,
b) einer thermischen Behandlung der Lösung, Suspension oder Aufschlämmung, welche die Molybdänausgangsverbindung und die Bismutausgangsverbindung die Eisenausgangsverbindungen, sowie die Zinkausgangsverbindungen und die Nickelausgangsverbindung enthält, in einer Behandlungszone mittels einer pulsierenden Strömung bei einer Temperatur von 200 bis 700 °C,
c) des Bildens eines nanokristallinen Bismut-Molybdänmischoxids,
d) des Ausbringens des in Schritt b) und c) erhaltenen nanokristallinen Bismut-Molybdänmischoxids aus dem Reaktor,
wobei die Molybdänausgangsverbindung ein Molybdat und die Bismutausgangsverbindung ein Bismutsalz ist.

Überraschenderweise wurde gefunden, dass durch das erfindungsgemäße Verfahren eine einheitliche Partikelgröße des erhaltenen Bismut-Molybdänmischoxids erhalten werden kann und eine Steuerung der Kristallisation, insbesondere hinsichtlich der Kristallitgröße, erreicht wird.

Das durch das erfindungsgemäße Verfahren erhaltene Bismut-Molybdänmischoxid zeichnet sich durch eine Kristallitgröße im Bereich von 10 nm bis 1000 nm, insbesondere von 10 nm bis 750 nm, bevorzugt 10 nm bis 500 nm und bevorzugter 10 nm bis 300 nm und weiter bevorzugt 10 nm bis 100 nm, insbesondere 10 nm bis 30 nm, aus.

Die katalytische Aktivität eines Katalysators, enthaltend das Bismut-Molybdänmischoxid, das durch das erfindungsgemäße Verfahren erhalten wurde, insbesondere bei einer Umsetzung von Propen zu Acrolein, konnte um bis zu 10%, verglichen mit Katalysatoren, die herkömmlich hergestelltes Bismut-Molybdänmischoxid enthalten, gesteigert werden.

Durch die hohe Aktivität und Selektivität eines derartigen Katalysators ist eine Herstellung von Acrolein aus Propen möglich. Auch eine Umsetzung von Isobuten zu Methacrolein oder analogen Verbindungen ist mit diesem Katalysator möglich. Insbesondere zeigt dieser Katalysator auch eine sehr gute Aktivität bei einer direkten Umsetzung von Propen oder Isobuten zu Acrylsäure bzw. Methacrylsäure, wobei die Umsetzung in einem Schritt erfolgen kann.

Als Molybdänausgangsverbindung wird ein Molybdat, besonders bevorzugt Ammoniumheptamolybdat-tetrahydrat verwendet. Jedoch ist dem Fachmann auf diesem Gebiet klar, dass auch andere im Stand der Technik bekannte Molybdate verwendet werden können.

Als Bismutausgangsverbindung wird ein Bismutsalz, wie Bismutchlorid, Bismutsulfat, Bismutacetat, Bismutoxid, besonders bevorzugt Bismutnitrat, eingesetzt.

Die Molybdänausgangsverbindung, die Bismutausgangsverbindung und die Eisenausgangsverbindung werden vorzugsweise zusammen als Lösung, Suspension oder Aufschlämmung eingesetzt. Am meisten ist bevorzugt, wenn die Ausgangsverbindungen als Lösung vorliegen. Um eine optimale Lösung zu erhalten, insbesondere bei schlecht lösliche Ausgangsverbindungen, kann die Lösung zusätzlich erwärmt werden, beispielsweise auf > 50 °C.

Erfindungsgemäß liegen noch weitere metallhaltige Ausgangsverbindungen zusammen mit der Molybdän-, Bismut- und Eisenausgangsverbindung in der Lösung, Suspension oder Aufschlämmung vor, nämlich eine Metallverbindungen von Nickel und eine Metallverbindung von Zink. Bevorzugt werden diese Metalle als Metallsalze, insbesondere als Oxide, Halogenide, Sulfat, Nitrate oder Acetate eingesetzt.

Weiter bevorzugt können auch Metallverbindungen oder Nichtmetallverbindungen, ausgewählt aus Li, Na, K, Rb, Cs, Mg, Ca, Ba, Sr, Ce, Mn, Cr, V, Nb, Se, Te, Sm, Gd, La, Y, Pd, Pt, Ru, Ag, Au, Si, Al, Tl, Zr, W und/oder P, zusätzlich verwendet werden.

Überraschenderweise wurde gefunden, dass das Verfahren bei relativ niedrigen Temperaturen von 200 bis 700 °C, bevorzugt 200 bis 500 °C, besonders bevorzugt von 250 bis 450 °C, besonders bevorzugt von 300 bis 400 °C durchgeführt werden kann. Bislang waren im Stand der Technik bevorzugte Temperaturen von mehr als 700 °C, und zwar bis zu 1400 °C bekannt. Ganz besonders überraschend wurde auch gefunden, dass durch das erfindungsgemäße Verfahren der Kristallisationsprozess des Bismut-Molybdänmischoxids gezielt gesteuert werden kann, insbesondere die Größe der Kristallite und die Porengrößenverteilung der entsprechenden Bismut-Molybdänmischoxide. Dies kann weiter durch die Verweilzeit in der Flamme bzw. durch die Reaktortemperatur vorteilhaft beeinflusst werden. Durch die pulsierende thermische Behandlung werden die entstehenden nanokristallinen Bismut-Molybdänmischoxidpartikel daran gehindert, zu agglomerieren. Typischerweise werden die nanokristallinen Partikel sofort durch den Strom an heißem Gas in eine kältere Zone überführt, wobei die Bismut-Molybdänmischoxidkristallite zum Teil mit Durchmessern von weniger als 20 nm erhalten werden.

Dies führt bei den so erhältlichen Bismut-Molybdänmischoxidkristalliten zu deutlich erhöhten BET-Oberflächen von > 1 m²/g, besonders bevorzugt 2 bis 50 m²/g und besonders bevorzugt 5 bis 35 m²/g. Die Bestimmung der BET-Oberfläche erfolgt nach DIN 66131 und 66132 (nach der Methode von Brunauer, Emmett und Teller).

In dem erfindungsgemäßen Verfahren können Suspensionen ohne zusätzliche Filtrations- und/oder Trocknungsschritte bzw. ohne Zugabe von zusätzlichen Lösungsmitteln innerhalb eines sehr kurzen Zeitraumes, typischerweise innerhalb weniger Millisekunden, bei vergleichsweise niedrigeren Temperaturen als bei Verfahren des Standes der Technik üblich, kalziniert werden. Die entstehenden Bismut-Molybdänmischoxid-Nanokristallite weisen hohe BET-Oberflächen auf. Damit kann ein Katalysator, enthaltend das durch das erfindungsgemäße Verfahren hergestellte Bismut-Molybdänmischoxid, ("Bismut-Molybdänmischoxidkatalysator") mit hoher Reaktivität, gutem Umsatz und guter Selektivität, insbesondere hinsichtlich einer Umsetzung von Propen zu Acrolein und/oder Acrylsäure oder Isobuten zu Methacrolein und/oder Methacrylsäure bereitgestellt werden.

Durch die annähernd gleiche Verweilzeit jedes Bismut-Molybdänmischoxidpartikels in dem durch das Verfahren erzeugten homogenen Temperaturfeld entsteht ein äußerst homogenes Endprodukt mit enger monomodaler Teilchenverteilung. Eine Vorrichtung zur Durchführung des erfindungsgemäßen Verfahrens bei der Herstellung derartiger monomodaler nanokristalliner Metalloxidpulver ist beispielsweise aus der DE 101 09 892 A1 bekannt. Im Gegensatz zu der dort beschriebenen Vorrichtung und dem dort offenbarten Verfahren benötigt das vorliegende Verfahren jedoch keinen vorgelagerten Verdampfungsschritt, in dem der Ausgangsstoff, d.h. die Molybdänausgangsverbindung, auf eine Verdampfungstemperatur erwärmt wird.

Die Molybdänausgangsverbindung, die Bismutausgangsverbindung, die Eisenausgangsverbindung, die Zinkausgangsverbindung und die Nickelausgangsverbindung aus der das erfindungsgemäße Bismut-Molybdänmischoxid hergestellt wird, werden direkt über ein Trägerfluid, insbesondere ein Trägergas, vorzugsweise ein inertes Trägergas, wie beispielsweise Stickstoff usw., in so genannte Reaktionskammern, d.h. in die Brennkammer, eingeführt. An die Reaktionskammer ist abgasseitig ein Resonanzrohr mit einem gegenüber der Reaktionskammer deutlich verringertem Strömungsquerschnitt angeschlossen. Der Brennkammerboden ist mit mehreren Ventilen zum Eintritt der Verbrennungsluft in die Brennkammer ausgestattet. Die aerodynamischen Ventile sind dabei strömungstechnisch und akustisch so mit der Brennkammer und der Resonanzrohrgeometrie abgestimmt, dass die in der Brennkammer erzeugten Druckwellen des homogenen "flammenlosen" Temperaturfeldes sich vorwiegend im Resonanzrohr pulsierend ausbreiten. Es bildet sich ein so genannter Helmholtzresonator mit pulsierender Strömung aus mit einer Pulsationsfrequenz zwischen 3 und 150 Hz, bevorzugt 5 bis 110 Hz.

Die Materialzufuhr in die Reaktionskammer erfolgt typischerweise entweder mit einem Injektor oder mit einer geeigneten Zweistoffdüse, Dreistoffdüse oder in einem Schenkdosierer.

Bevorzugt werden die Molybdänausgangsverbindung, die Bismutausgangsverbindung, die Eisenausgangsverbindung, die Zinkausgangsverbindung und die Nickelausgangsverbindung in verdüster Form in die Reaktionskammer eingebracht, so dass eine feine Verteilung im Bereich der Behandlungszonen gewährleistet ist.
Nach der thermischen Behandlung werden die entstandenen nanokristallinen Bismut-Molybdänmischoxide, wenn möglich mittels des Trägerfluids, sofort in eine kältere Zone der Reaktionskammer überführt, sodass sie in der kälteren Zone abgeschieden und ausgetragen werden können. Die Ausbeute des erfindungsgemäßen Verfahrens beträgt nahezu 100%, da das entstehende Produkt vollständig aus dem Reaktor ausgetragen werden kann.
Typischerweise wird das Verfahren bei einem Druck im Bereich von Normaldruck bis 40 bar durchgeführt.
Es wurde gefunden, dass das durch das erfindungsgemäße Verfahren erhaltene nanokristalline Bismut-Molybdänmischoxid bevorzugt eine Kristallitgröße im Bereich von 5 nm bis 1000 nm, bevorzugt von 10 nm bis 800 nm, ganz besonders bevorzugt 15 bis 550 nm aufweist, was, wie vorstehend schon ausgeführt, durch die Pulsation der thermischen Behandlung bevorzugt eingestellt werden kann. Die Bestimmung der Teilchengröße kann durch dem Fachmann bekannte Verfahren, wie XRD oder TEM erfolgen.
Weiterhin werden durch das erfindungsgemäße Verfahren Bismut-Molybdänoxidpartikel erhalten, die eine BET-Oberfläche von vorzugsweise > 1 m²/g, besonders bevorzugt 2 bis 50 m²/g und ganz besonders bevorzugt 5 bis 35 m²/g besitzen.

Das durch das erfindungsgemäße Verfahren erhaltene Molybdänmischoxid eignet sich zur Verwendung als katalytisch aktive Komponente eines Katalysators, beispielsweise eines Katalysators zur Umsetzung von Propen zu Acrolein und/oder Acrylsäure oder Isobuten zu Methacrolein und/oder Methacrylsäure. Insbesondere eignet sich ein so erhaltener Katalysator auch für eine direkte Umsetzung von Propen oder Isobuten zu Acrylsäure bzw. Methacrylsäure, wobei die Umsetzung in einem Schritt erfolgen kann.

Das durch das erfindungsgemäße Verfahren hergestellte Bismut-Molybdänmischoxid kann zusammen mit einem geeigneten Bindemittel zu einem Extrudat (Tabletten, Formkörper, Honigwabenkörper und dergleichen) verarbeitet werden. Als Bindemittel kann jedes dem Fachmann geläufige und geeignet erscheinende Bindemittel verwendet werden, insbesondere Silicatmaterialien, Aluminiumoxid, Zirkoniumverbindungen, Titanoxid, sowie deren Mischungen, und Materialien, wie z.B. Zement, Ton, Silica/Alumina. Bevorzugte Bindemittel sind unter anderem Pseudoboehmit sowie silicatische Bindemittel wie kolloidales Siliciumoxid oder Silicasol.

Das durch das erfindungsgemäße Verfahren hergestellte Bismut-Molybdänmischoxid kann ferner zusammen mit anderen Komponenten, vorzugsweise mit einem Bindemittel, besonders bevorzugt mit einem organischen Bindemittel, beispielsweise organische Kleber, Polymere, Harze oder Wachse, zu einem Washcoat verarbeitet werden, der auf einen metallischen oder keramischen Träger aufgebracht werden kann. Gegebenenfalls können zusätzliche Imprägnierschritte oder Kalzinierschritte erfolgen.

Bevorzugt liegt das durch das erfindungsgemäße Verfahren hergestellte Bismut-Molybdänmischoxid als Beschichtung auf einem Träger vor. Bevorzugtes Trägermaterial ist Steatit, besonders bevorzugt sind Steatitkugeln. Zur Durchführung der Beschichtung wird bevorzugt eine sogenannte Wirbelbett-Coating-Vorrichtung verwendet.

Die Erfindung wird anhand der nachstehenden und nicht als einschränkend zu verstehenden Ausführungsbeispiele und Figuren näher erläutert. Die verwendete Vorrichtung entspricht dabei weitgehend der in der DE 101 09 892 A1 beschrieben Vorrichtung, mit dem Unterschied, dass die zur Durchführung des erfindungsgemäßen Verfahrens verwendete Vorrichtung keine Verdampfervorstufe aufwies.
Figur 1 zeigt die Korngrößenverteilung der Suspension aus Beispiel 2.
Figur 2 zeigt das XRD-Spektrum des hergestellten Bismut-Molybdänmischoxids, das bei 450 °C erhalten wurde.
Figur 3 zeigt die Korngrößenverteilung der Suspension aus Beispiel 3.
Figur 4 zeigt das XRD-Spektrum des hergestellten Bismut-Molybdänmischoxids, das bei 500°C erhalten wurde.
Figur 5 zeigt die Korngrößenverteilung der Suspension aus Beispiel 4.
Figur 6 zeigt die Korngrößenverteilung der Suspension aus Beispiel 5.
Figur 7 zeigt das XRD-Spektrum des bei 600 °C erhaltenen Bismut-Molybdänmischoxids.

### Ausführungsbeispiele:

### Allgemeines:

Die wesentlichen Vorteile der Präparation mit Hilfe des Pulsationsreaktors liegen in der Verkürzung der gesamten Präparationszeit, des geringen Aufwandes (benötigt wird nur der Reaktor) und darin, dass Trocknung und Aufbereitung des Produktes entfallen. Durch den Pulsationsreaktor lassen sich in einem Schritt die gewünschten BET-Oberflächen, Partikelgrößen und auch die Kristallinität des Materials variieren.

### Beispiel 1: Vergleichsbeispiel (gemäß DE 10 2006 015710 A1)

Eine Lösung I wurde hergestellt, indem Nitrate von Eisen, Cobalt, Nickel, Mangan, Kalium in den Massenanteilen 23,2:47,26:29,28:0,0646:0,2067 in 3,5 Liter Wasser gelöst, unter Rühren auf 40 °C erhitzt und eine salpetersaure Lösung aus 0,1 mol Sm³⁺ und 2 mol HNO₃ zugegeben wurde.

Für eine Lösung II wurde bei 40 °C eine Lösung von 2118,6 g Ammoniumheptamolybdat in 2,7 l Wasser bereitet, dazu wurde 4,4 g Phosphorsäure sowie 0,428 g Aerosil 200 (Degussa) und 14 g Aluminiumoxid in 1 l Wasser gegeben.

Lösung II wurde langsam und unter intensivem Rühren zu Lösung I gegeben. In einem getrennten Gefäß wurde eine Lösung III, bestehend aus 790 g Bismutnitrat und 0,72 mol HNO₃ angesetzt. Durch Zugabe dieser Lösung zu den anderen Aktivkomponenten wurde das Kopräzipitat für die Herstellung der aktiven Katalysatorphase erhalten.

Das Kopräzipitat wurde 12 Stunden intensiv gerührt. Die erhaltene Suspension wurde in einem Sprühtrockner mit Drehscheibe bei einer Gaseintrittstemperatur von 350 °C getrocknet. Die Luftmenge wurde so eingestellt, dass eine Austrittstemperatur von 100 +/- 10 °C erhalten wurde.

Der erhaltene mittlere Partikeldurchmesser des so hergestellten Pulvers betrug 55 µm. Dieses Pulver wurde in einem Umluftofen bei einer Temperatur von 445 °C für 1 Stunde behandelt, bis sich ein Mischoxid bildete, das im nächsten Schritt auf einen mittleren Partikeldurchmesser von 1 µm gemahlen wurde. Das Mischoxid wurde als wässrige Suspension durch eine Zweistoffdüse auf einen keramischen kugelförmigen Katalysatorträger gesprüht und bei 60 °C im Luftstrom getrocknet. Zur Homogenisierung der Pellets wurden diese in einer Trommel umgewälzt. Zur Verfestigung der aufgebrachten Aktivmasse wurde das erhaltene Gut auf 540 °C für 1 Stunde erwärmt.

Der so hergestellte Katalysator wies die Zusammensetzung (Mo₁₂Bi_{1,5}(Co+Ni)_{8,0}Fe_{1,8}Mn_{0,1}K_{0,06}P_{0,04}Al₂₇₅Si_{0,66}Sm_{0,1})Oₓ auf.

### Test des Katalysators:

Der Katalysator des Beispiels 1 wurde mit einem Gemisch der Zusammensetzung von 7,5 Vol.-% Propen (chemical grade), 58 Vol.-% Luft, und Inertgas (insgesamt 100 Vol.-%) beschickt. Der Gesamtgasstrom betrug 36,9 l/min. Die Temperatur des Wärmeträgers betrug 365 °C. Der Umsatz des Propens lag bei 89,5 mol-%, dabei betrug die Produktselektivität an Acrolein und Acrylsäure 95,3 % (siehe auch Beispiel 7, Tabelle 1).

### Beispiel 2 (erfindungsgemäß)

In ein 100 1-Gefäß wurden 60 l VE-Wasser (VE = vollentsalzt) eingewogen. Dieses wurde auf 50 °C aufgeheizt. Dem VE-Wasser wurden folgende Stoffe nacheinander zugegeben:

| Stoff | Menge [g] | Farbe Lösung nach Zugabe |
|---|---|---|
| Nickelnitratlösung (Ni: 12%) | 6599,25 | grün |
| Eisennitrat x 9H₂O | 1664,68 | grün |
| Zinknitrat x 6H₂O | 1202,67 | grün |
| Ammoniumheptamolybdat x 4H₂O | 4344,48 | grün-gelb |

Die Suspension wurde unter Rühren auf 75 °C erwärmt. Die Suspension wurde auf 64 °C abgekühlt und dann 3290 g Ludox® AS40 zugegeben. Nun wurde die Bismutnitratlösung (2,5 l 10%-ige Salpetersäure + 991,04 g Bismutnitrat x 5H₂O) bei einer Temperatur von 62 °C zugegeben. Die Suspension hatte nun eine Temperatur von 58 °C und wurde für 10 Minuten gerührt.

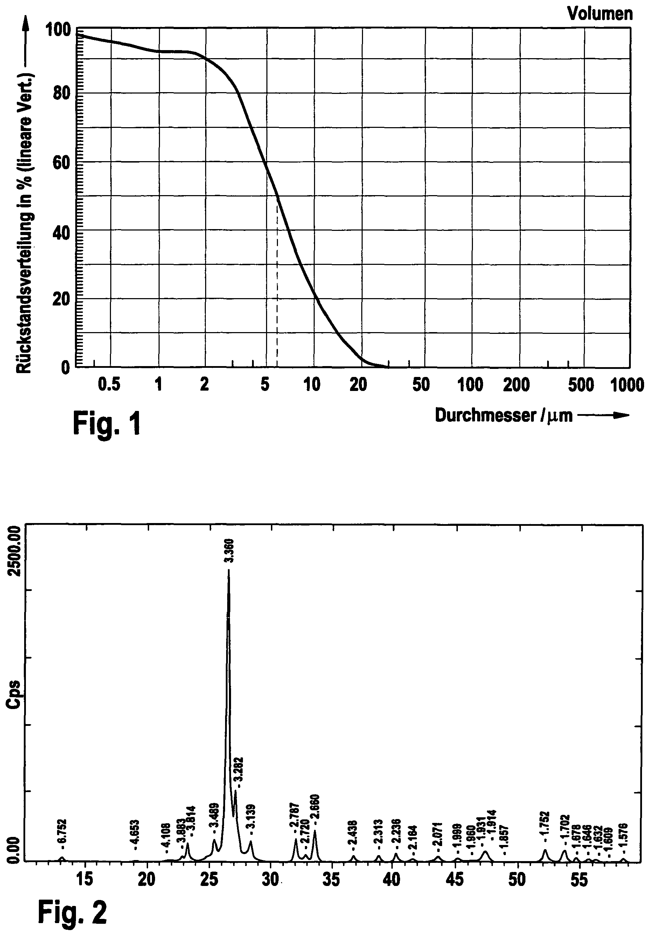

Die Korngrößenverteilung der Suspension wurde bestimmt: Die Ergebnisse sind in Figur 1 zu sehen

Die Eindüsung der Suspension in den Pulsationsreaktor erfolgte mit einer Dosierrate von 20 kg/h. Die Temperatur betrug 450 °C. Das XRD-Spektrum der erhaltenen Proben zeigt folgendes Ergebnis:
Das bei 450 °C im Pulsationsreaktor hergestellte Material wie Katalysator hat eine BET-Oberfläche von 23 m²/g auf und ist durch das in Figur 2 gezeigte Pulverdiffraktogramm (XRD) charakterisiert.

Aus dem XRD Diffraktogramm lässt sich die Zersetzung der Nitratprecursoren sowie die Ausbildung von Metall-Molybdat Phasen wie z.B. Bismutmolybdat ableiten.

Für die unten beschriebenen Coatingversuche wurden 1,45 kg des bei 450 °C im Pulsationsreaktor verdüsten Materials hergestellt.

### Beispiel 3 (erfindungsgemäß)

In ein 100 1-Gefäß wurden 60 l VE-Wasser eingewogen. Dieses wurde auf 50 °C aufgeheizt. Dem VE-Wasser wurden folgende Verbindungen nacheinander zugegeben:

| Stoff | Menge [g] | Farbe Lösung nach Zugabe |
|---|---|---|
| Nickelnitratlösung (Ni: 12%) | 6599,25 | grün |
| Eisennitrat x 9H₂O | 1664,68 | grün |
| Zinknitrat x 6H₂O | 1202,67 | grün |
| Ammoniumheptamolybdat x 4H₂O | 4344,48 | grün-gelb |

Die Suspension wurde unter Rühren auf 75 °C erwärmt. Die Suspension wurde auf 64 °C abgekühlt und dann 3290 g Ludox® AS40 zugegeben. Nun wurde die Bismutnitratlösung (2,5 l 10%-ige Salpetersäure + 991,04 g Bismutnitrat x 5H₂O) bei einer Temperatur von 62 °C zugegeben. Die Suspension hatte nun eine Temperatur von 58 °C und wurde für 10 Minuten gerührt.

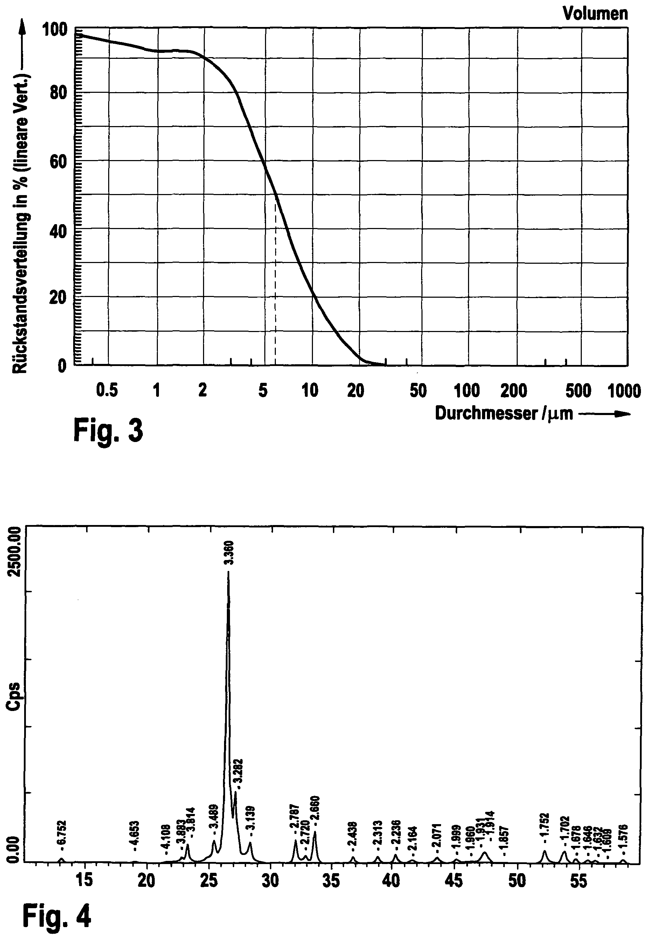

Von der Suspension wurde die Korngrößenverteilung bestimmt. Die Ergebnisse sind der Figur 3 zu entnehmen.

Die Eindüsung der Suspension in den Pulsationsreaktor erfolgte mit einer Dosierrate von 20 kg/h.

Das erhaltene Material wurde bei 500 °C im Pulsationsreaktor verdüst. Das XRD-Diffraktogramm der erhaltenen Proben zeigt folgendes Ergebnis:
Das bei 500 °C im Pulsationsreaktor erzeugte Material hat eine BET-Oberfläche von 21 m²/g und ist durch das in Figur 4 gezeigte Diffraktogramm charakterisiert.

Aus dem XRD-Diffraktogramm lässt sich die Zersetzung der Nitratprecursoren sowie die Ausbildung von Metall-Molybdat Phasen wie z.B. Bismutmolybdat ableiten. Die Hauptfraktion besteht auf Grund des Bi-Unterschusses aus Eisenmolybdat in allen Fällen der untersuchten Katalysatoren.

Für die unten beschriebenen Beschichtungsversuche wurden 1,45 kg des bei 500 °C im Pulsationsreaktor verdüsten Pulvers hergestellt.

### Beispiel 4 (erfindungsgemäß)

In ein 100 1-Gefäß wurden 60 l VE-Wasser eingewogen. Dieses wurde auf 50 °C aufgeheizt. Dem VE-Wasser wurden folgende Stoffe nacheinander zugegeben:

| Stoff | Menge [g] | Farbe Lösung nach Zugabe |
|---|---|---|
| Nickelnitratlösung (Ni: 12%) | 6599,25 | grün |
| Eisennitrat x 9H₂O | 1664,68 | grün |
| Zinknitrat x 6H₂O | 1202,67 | grün |
| Ammoniumheptamolybdat x 4H₂O | 4344,48 | grün-gelb |

Die Suspension wurde unter Rühren auf 75 °C erwärmt. Die Suspension wurde auf 64 °C abgekühlt und dann 3290 g Ludox® AS40 zugegeben. Nun wurde die Bismutnitratlösung (2,5 l 10%-ige Salpetersäure + 991,04 g Bismutnitrat x 5H₂O) bei einer Temperatur von 62 °C zugegeben. Die Suspension hatte nun eine Temperatur von 58 °C und wurde für 10 Minuten gerührt.

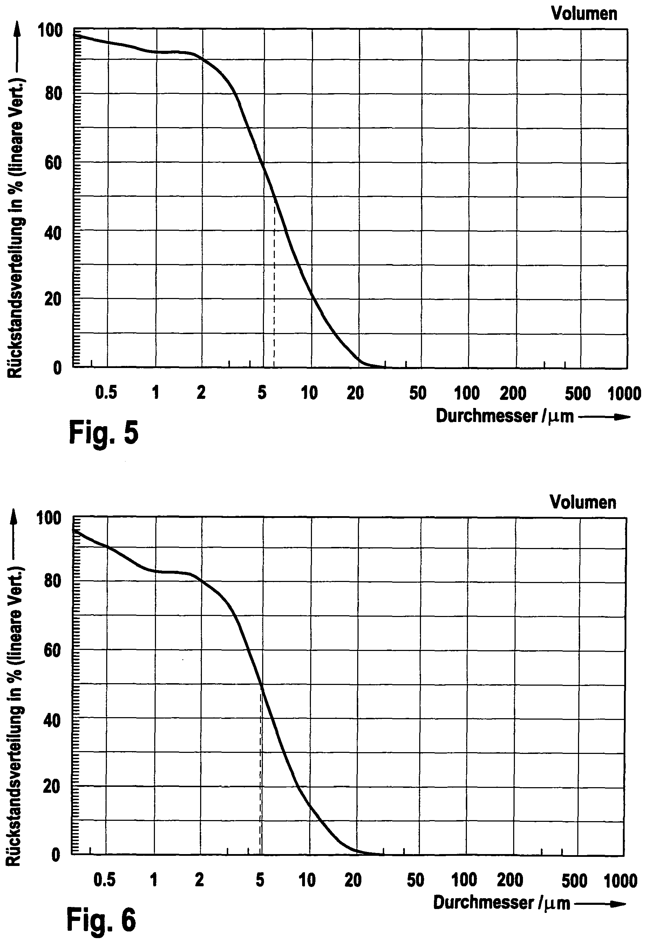

Von der Suspension wurde die Korngrößenverteilung bestimmt. Die Ergebnisse sind der Figur 5 zu entnehmen.

Die Eindüsung der Suspension in den Pulsationsreaktor erfolgte mit einer Dosierrate von 20 kg/h. Das erhaltene Material wurde bei 600 °C im Pulsationsreaktor verdüst.

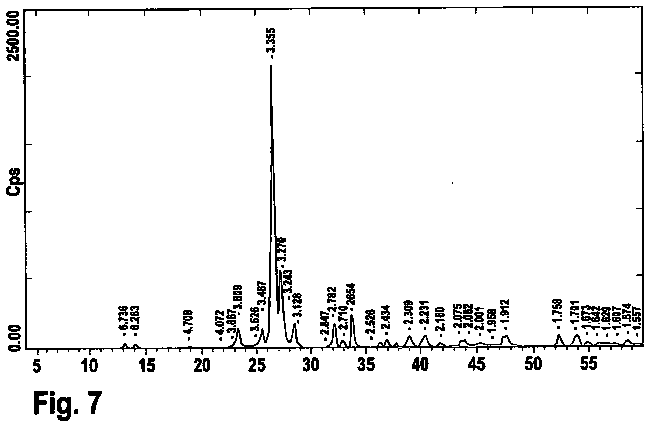

Das bei 600 °C im Pulsationsreaktor erzeugte Material wies eine BET Oberfläche von 18 m²/g auf und ist durch das in Figur 7 dargestellte Diffraktogramm charakterisiert.

Die Zersetzung der Nitratprecursoren und die Bildung einer BiMoO₄ Phase kann aus dem XRD-Diffraktogramm abgeleitet werden.

Für die unten beschriebenen Coatingversuche wurden 1,45 kg des bei 600 °C im Pulsationsreaktor verdüsten Pulvers hergestellt.

### Beispiel 5 (Referenzbeispiel)

1. Lösung: In ein 100 1-Gefäß wurden 40 l VE-Wasser eingewogen. Dieses wurde auf 55 °C aufgeheizt. Dem VE-Wasser wurden folgende Stoffe nacheinander zugegeben.

| Stoff | Menge [g] | Temperatur [°C] | ph-Wert | Farbe Lösung nach Zugabe |
|---|---|---|---|---|
| Eisennitrat x 9H₂O | 587,12 | 55 | 2,73 | orange |
| Nickelnitratlösung (Ni: 12%) | 7108,15 | 50 | 1,92 | grün |
| Cobaltnitrat x 6H₂O | 563,93 | 50 | 1,84 | grün |
| Kaliumnitrat | 12,24 | 50 / 30** | 1,84 | grün |
| LUDOX AS40 | 3638,04g | 28** | 1,92 | dunkelgrün |

Das LUDOX® AS40 wurde erst am nächsten Tag zugegeben. Deswegen war die Lösung auf 30 °C abgekühlt.
2. Lösung: In ein 100 l-Blechfass wurden 20 l VE-Wasser vorgelegt und danach auf 57 °C aufgeheizt (pH-Wert: 5,00). Dem VE-Wasser wurden folgende Stoffe nacheinander zugegeben:

| Stoff | Menge [g] | Temperatu r [°C] | ph-Wert | Farbe Lösung nach Zugabe |
|---|---|---|---|---|
| Ammoniumheptamolybda t x 4H₂O | 5131,56 | 50 | 5,46 | farblos/ grünlich |
| Phosphorsäure | 280,09 | 50 | 5,42 | hellgrün |

3. Lösung: In einen 10l-Gefäß wurden 2115,4 g VE-Wasser (20°C) eingewogen.

| Stoff | Menge [g] | Temperatur [°C] | ph-Wert | Farbe Lösung nach Zugabe |
|---|---|---|---|---|
| Salpetersäure (65%-ig) | 384,6 | 28 | --- | farblos |
| Bismutnitrat x 5H2O | 1174,90g | 25 | --- | farblos |

Die 1. Lösung wurde mit der 2. Lösung unter intensivem Mischen miteinander vereinigt (Temperatur: 34 °C; pH-Wert: 4,76). Danach lag eine hellgrüne Suspension vor. Anschließend wurde die 3. Lösung zugegeben (Temperatur: 34 °C; pH-Wert: 2,02). Die Suspension (ocker/gelb) wurde für eine Stunde gerührt. Dabei konnte eine leichte Gasentwicklung beobachtet werden.

Die Korngrößenverteilung der Suspension wurde bestimmt. Die Ergebnisse sind der Figur 6 zu entnehmen.

Die Eindüsung der Suspension in den Pulsationsreaktor erfolgte mit einer Dosierrate von 20 kg/h.

Mit den oben genannten Parametern wurde bei 600 °C, die Suspension eingedüst. Aus dem nicht gezeigten XRD ist die Bildung einer BiMoO₄ Phase ersichtlich. Das XRD Spektrum ist im Wesentlichen identisch mit dem in Figur 7 gezeigten XRD Spektrum zu Beispiel 4.

Für Beschichtungsversuche standen 2,06 kg des bei 600 °C und 1,07 kg des bei 400 °C im Pulsationsreaktor verdüsten Pulvers zur Verfügung.

### Beispiel 6 (Herstellung der beschichteten Katalysatoren)

Zur Durchführung der Beschichtung kam eine Wirbelbett-Coating-Vorrichtung zum Einsatz.

Die Beschichtungen der Steatit-Kugeln mit den verschiedenen Bismut-Molybdänmischoxid-Aktivmassen aus Beispiel 1 bis 4 erfolgten unter folgenden Bedingungen:
Es wurden 22,22 g des Materials in einen Messzylinder eingewogen, mit 500 ml destilliertem H₂O aufgeschlämmt. Die entstandene Suspension wird intensiv gerührt. Anschließend wurde 8,89 g Binder zugegeben und 1 Stunde lang auf einem Magnetrührer gerührt.

Das Coating der hergestellten Suspension erfolgte auf einer Einwaage von 80 g Steatitkugeln mit (2 - 4 mm), wobei die Aktivmassenbeladung 20% betrug (50 g Pulver per 200 g Steatitkugeln) . Der Katalysator wurde dann bei 110 °C in Luft getrocknet.

### Beispiel 7 (Ermittlung der katalytischen Leistungsdaten der Katalysatoren)

In ein 120 cm langes Reaktionsrohr mit einem Innendurchmesser von 24,8 mm wurden 21 g Katalysator, verdünnt mit 350 g Steatitkugeln (4,5 mm Durchmesser) zur Vermeidung von Hotspots, auf einer Länge von 105 cm eingefüllt. Das Reaktionsrohr befand sich in einer flüssigen Salzschmelze die auf Temperaturen bis 500 °C aufgeheizt werden kann. In der Katalysatorschüttung befand sich ein 3 mm Schutzrohr mit eingebautem Thermoelement über das die Katalysatortemperatur über die komplette Katalysatorkombination angezeigt werden kann. Zur Ermittlung der katalytischen Leistungsdaten wurden über den Katalysator 7,5 vol.-% Propen, 58 vol.-% Luft und Stickstoff (insgesamt 100 vol.-%) bei maximal 4500 Nl/h geleitet. Der Propenumsatz und die Acroleinselektivität wurde bei einer mittleren Katalysatortemperatur von 365 °C eingestellt, und das Reaktionsgas nach Reaktionsrohraustritt auf seine Bestandteile hin analysiert. Die Ergebnisse der Tests mit den in den Beispielen 1 bis 4 erhaltenen Materialien als Katalysator (hergestellt gemäß Beispiel 6) sind in Tabelle 1 aufgeführt.

**Tabelle 1: Leistungsdaten erfindungsgemäßer Katalysatoren und eines Vergleichskatalysators**

| | Beispiel 1 (Vergleichsbeispiel) | Beispiel 2 (erfindungsgemäß) | Beispiel 3 (erfindungsgemäß) | Beispiel 4 (erfindungsgemäß) |
|---|---|---|---|---|
| Propenumsatz | 89,5 | 91 | 93,5 | 94 |
| Acroleinselektivität | 95,3 | 96,5 | 96,5 | 96 |
| **Ausbeute** | **85,2** | **87,81** | **90,3** | **90,3** |

Wie aus Tabelle 1 zu erkennen ist, liegen die Vorteile der erfindungsgemäßen Katalysatoren in einer höheren Ausbeute und Acroleinselektivität als bei einem Katalysator, bei dem die Bismut-Molybdänmischung Aktivmasse nach einem Verfahren des Standes der Technik hergestellt wurde (Vergleichsbeispiel, Beispiel 1).

### Zusammenfassung:

Die Erfindung betrifft ein Verfahren zur Herstellung eines nanokristallinen Bismut-Molybdänmischoxids, umfassend die Schritte
a) des Einbringens einer Lösung, Suspension oder Aufschlämmung, enthaltend eine Molybdänausgangsverbindung und eine Bismutausgangsverbindung, sowie eine Nickelausgangsverbindungen und eine Zinkausgangsverbindungen, in eine Reaktionskammer mittels eines Trägerfluids,
b) einer thermischen Behandlung der Lösung, Suspension oder Aufschlämmung, welche die Molybdänausgangsverbindung und die Bismutausgangsverbindung, sowie die Zinkausgangsverbindung und die Nickelausgangsverbindung enthält, in einer Behandlungszone mittels einer pulsierenden Strömung bei einer Temperatur von 200 bis 700 °C,
c) des Bildens eines nanokristallinen Bismut-Molybdänmischoxids,
d) des Ausbringens des in Schritt b) und c) erhaltenen nanokristallinen Bismut-Molybdänmischoxids aus dem Reaktor,
wobei die Molybdänausgangsverbindung ein Molybdat und die Bismutausgangsverbindung ein Bismutsalz ist.

## Patentansprüche

1. Verfahren zur Herstellung eines nanokristallinen Bismut-Molybdänmischoxids umfassend die Schritte
a) des Einbringens einer Lösung, Suspension oder Aufschlämmung, enthaltend eine Molybdänausgangsverbindung, eine Bismutausgangsverbindung, eine Eisenausgangsverbindung, sowie eine Nickelausgangsverbindung und eine Zinkausgangsverbindung, in eine Reaktionskammer mittels eines Trägerfluids,
b) einer thermischen Behandlung der Lösung, Suspension oder Aufschlämmung, welche die Molybdänausgangsverbindung, die Bismutausgangsverbindung, die Eisenausgangsverbindung sowie die Zinkausgangsverbindung und die NickelAusgangsverbindung enthält, in einer Behandlungszone mittels einer pulsierenden Strömung bei einer Temperatur von 200 bis 700 °C,
c) des Bildens eines nanokristallinen Bismut-Molybdänmischoxids,
d) des Ausbringens des in Schritt b) und c) erhaltenen nanokristallinen Bismut-Molybdänmischoxids aus dem Reaktor,
wobei die Molybdänausgangsverbindung ein Molybdat und die Bismutausgangsverbindung ein Bismutsalz ist.

2. Verfahren nach Anspruch 1, **dadurch kennzeichnet, dass** die Molybdänausgangsverbindung Ammoniumheptamolybdat-tetrahydrat ist.

3. Verfahren nach Anspruch 1 oder 2, **dadurch gekennzeichnet, dass**, die Bismutausgangsverbindung Bismutnitrat ist.

4. Verfahren nach einem der Ansprüche 1 bis 3, **dadurch gekennzeichnet, dass** das Trägerfluid ein Gas ist.

## Claims

1. Process for preparing a nanocrystalline bismuthmolybdenum mixed oxide, which comprises the steps
a) introduction of a solution, suspension or slurry containing a molybdenum starting compound, a bismuth starting compound, and an iron starting compound and also a nickel starting compound and a zinc starting compound into a reaction chamber by means of a carrier fluid,
b) thermal treatment of the solution, suspension or slurry containing the molybdenum starting compound, the bismuth starting compound, the iron starting compound and also the zinc starting compound and the nickel starting compound in a treatment zone by means of a pulsating flow at a temperature of from 200 to 700°C,
c) formation of a nanocrystalline bismuthmolybdenum mixed oxide,
d) discharge of the nanocrystalline bismuthmolybdenum mixed oxide obtained in step b) and c) from the reactor,
where the molybdenum starting compound is a molybdate and the bismuth starting compound is a bismuth salt.

2. Process according to Claim 1, **characterized in that** the molybdenum starting compound is ammonium heptamolybdate tetrahydrate.

3. Process according to Claim 1 or 2, **characterized in that** the bismuth starting compound is bismuth nitrate.

4. Process according to any of Claims 1 to 3, **characterized in that** the carrier fluid is a gas.

## Revendications

1. Procédé de fabrication d'un oxyde mixte de bismuth et de molybdène nanocristallin comprenant les étapes suivantes :
a) l'introduction d'une solution, d'une suspension ou d'une suspension épaisse, contenant un composé de départ de molybdène, un composé de départ de bismuth, un composé de départ de fer, ainsi qu'un composé de départ de nickel et un composé de départ de zinc dans une chambre de réaction au moyen d'un fluide vecteur,
b) un traitement thermique de la solution, de la suspension ou de la suspension épaisse, qui contient le composé de départ de molybdène, le composé de départ de bismuth, le composé de départ de fer, ainsi que le composé de départ de zinc et le composé de départ de nickel, dans une zone de traitement au moyen d'un courant pulsé à une température de 200 à 700 °C,
c) la formation d'un oxyde mixte de bismuth et de molybdène nanocristallin,
d) le déchargement de l'oxyde mixte de bismuth et de molybdène nanocristallin obtenu aux étapes b) et c) du réacteur,
le composé de départ de molybdène étant un molybdate et le composé de départ de bismuth étant un sel de bismuth.

2. Procédé selon la revendication 1, **caractérisé en ce que** le composé de départ de molybdène est le tétrahydrate d'heptamolybdate d'ammonium.

3. Procédé selon la revendication 1 ou 2, **caractérisé en ce que** le composé de départ de bismuth est le nitrate de bismuth.

4. Procédé selon l'une quelconque des revendications 1 à 3, **caractérisé en ce que** le fluide vecteur est un gaz.
